# EUROPEAN PATENT SPECIFICATION

(11) **EP 2 851 068 B1**
(45) Date of publication and mention of the grant of the patent: **23.10.2019**
(21) Application number: 14199017.6
(22) Date of filing: 07.09.2006
(51) Int. Cl.: A61K 9/16, A61K 9/20, A61K 31/67, A61K 31/192, A61K 45/06, A61P 29/00

(54) **Composition comprising a NSAID and paracetamol**
Zusammensetzung mit einem NSAR und Paracetamol
Composition comprenant un AINS et du paracetamol

(30) Priority: 22.09.2005 GB 0519350
(43) Date of publication of application: 25.03.2015
(62) Divisional of application: 06779316.6
(73) Proprietor: Reckitt Benckiser Healthcare (UK) Limited, Slough, Berkshire SL1 3UH (GB)
(72) Inventor: Sherry, Robert, Hull, East Yorkshire HU8 7DS (GB)
(74) Representative: Hodgetts, Catherine Dawn

(56) References cited:
- WO-A-2006/016126
- WO-A-2006/043025
- US-B1- 6 787 157

## Description

The present invention relates to compositions containing a non-steroidal anti-inflammatory drug, to processes to prepare them and uses thereof.

Non-steroidal anti-inflammatory drugs (NSAIDs) are a widely used class of medicaments. They are a well defined group of compounds and include phenylpropionic acids such as ibuprofen, naproxen, ketoprofen and flurbiprofen. They are primarily used for the treatment of one or more of pain, inflammation and fever, for example rheumatoid arthritis, ankylosing spondylitis, osteoarthritis, post-operative pain, post-partum pain and soft tissue injuries.

NSAIDs are generally acidic and substantially insoluble drugs. They are conveniently administered as an oral pharmaceutical composition in the form of tablets. Thus pharmaceutically acceptable excipients must be chosen for combination with the NSAID, with which the NSAID is compatible and with which it can form tablets having a satisfactory hardness and also release the medicament rapidly into the body so that it is available for absorption.

A major issue in connection with the disorders identified above is to improve the onset of action of the NSAID, particularly in the treatment of pain. It is believed that rapid disintegration of a formulation releases the drug into the body quickly leading to a more rapid onset of therapeutic action compared with a standard dosage form. Accordingly, it is desired to produce a solid dosage form for oral administration adapted to disintegrate quickly in the gastro-intestinal tract. However, as many of the NSAIDs are acidic drugs, accordingly, absorption can be a problem in the acidic conditions encountered in the stomach. Furthermore, although the literature has proposed many formulations adapted to disintegrate quickly, a major problem occurs with ibuprofen and other NSAIDs as they may need to be administered in relatively high doses, e.g. up to 800 mg per unit dose. Thus, there is a problem to provide a dosage form which includes the NSAID together with excipients useful to formulate the tablet into the dosage form and also excipients useful to ensure rapid disintegration, but not to provide a tablet that is too large for patient consumption or cannot be produced according to standard large scale manufacturing processes. Furthermore, the solid dosage form must be sufficiently hard to withstand the rigours of the manufacturing process (for example as encountered during the stage of film coating in a perforated rotating drum and packaging etc) but must have appropriate disintegration characteristics to ensure rapid release of the drug from the formulation and also appropriate dissolution characteristics. Another significant problem that must be overcome is to ensure that the composition is capable of being compressed with standard tabletting machinery without sticking to the punches of the tabletting machine.

European Patent Application No. 362728 (1990) relates to a procedure for obtaining ibuprofen powder that flows easily and has improved storage and formulation properties for direct tabletting. It is said that hardening molten ibuprofen is extremely problematic as the molten ibuprofen that congeals at about 74.8°C becomes oily and viscous as it cools, tends to be under-cooled and can be transformed into a crystalline state only very slowly. In accordance with EP 362728, it is proposed that ibuprofen particles are obtained through the solidification of molten ibuprofen on a contact cooler (e.g. a roller or cooling belt) at 0-50°C, using seeding, followed by crushing. It is said that seeding can be carried out by coating with a molten layer that congeals on the contact cooler. The seed can also be added to the roller in particle form or worked into the molten product. It is said that any process to prepare molten ibuprofen can be used to manufacture the ibuprofen particles, although, the procedure is of particular relevance for a molten ibuprofen product obtained through the rectification of ibuprofen in accordance with German Patent Application No 3802619. The single illustrative Example shows that the ibuprofen powder produced by the process requires to be mixed with a significant number of additional excipients prior to tabletting.

European Patent Application 686392 (1995) relates to a thermal process for the production of directly tablettable granules comprising melt-extruding an active compound having a low melting point and necessary tablet auxiliaries at elevated temperature to give a homogeneous non-agglutinating extrudate which is then comminuted to give tablettable granules. By means of the mixing and kneading elements of the extruder, the mixture is compacted to give an extrudate at a temperature at which a part of the active compound is melted.

The extrudate is pressed through a perforated plate to give thin strands of 0.3-2.0 mm diameter and comminuted after cooling to the desired particle size of the granules. The granules thus obtained can immediately be subjected to tabletting, only a lubricant being required. The active is thus present as unmelted crystals within a solidified melt of the active. It is said that by means of the process, all auxiliaries such as binder, disintegration auxiliaries, fillers and other auxiliaries can be incorporated directly in the granules.

In this respect, WO 01/41733 by The Boots Company PLC discloses that if a disintegrating agent is incorporated into a molten NSAID and intimately combined therewith and then is cooled and milled to produce a granule, a composition capable of tabletting with minimum tabletting excipients and having advantageous tabletting, disintegration and dissolution properties is provided, if silicon dioxide is incorporated therein. This method was substantially improved upon as disclosed in WO 02/098391. The NSAID was formed as a homogenous extrudate in a melt-extrusion process. Preferred cooling and processing conditions are described.

All of the methods described above relate to the manufacture of tabletting compositions comprising an NSAID as the only major therapeutic agent. Whilst the presence of other agents is not excluded, the inventions are all primarily concerned with the production of NSAIDs.

US6787157 discloses solid formulations comprising ibuprofen and paracetamol.

It may be desirable to provide a pharmaceutical composition which includes a further analgesic active agent in addition to the NSAID i.e. a combination product. One such analgesic agent which has been combined with NSAIDs is acetaminophen (also known as paracetamol) which displays analgesic (pain relief) and antipyretic (fever relief) properties, but essentially no anti-inflammatory action. Such products are, amongst other things, extremely effective for treating pain and fever i.e. headache, muscle and joint pain, back ache, cough, cold and flu. However, there are further problems associated with providing an oral dosage form (i.e. tablet) containing paracetamol.

In particular, paracetamol is a fine soft powder, and as such, it does not lend itself for compressing directly into tablets. Thus, it is necessary to pre-treat the paracetamol prior to compression into a solid dosage form. Typically, the paracetamol is subjected to a wet granulation process to produce a granular form of paracetamol which is subsequently mixed with additional tabletting excipients and the resultant mixture compressed to form tablets. Alternatively, direct compression forms of paracetamol, which have typically been subjected to a pre-treatment process, may be obtained from specialist suppliers. These pre-treatment requirements typically increase the complexity and cost associated with processes for producing solid dosage forms of paracetamol. Moreover, paracetamol is typically administered in relatively high doses e.g. up to 500 mg per unit dose. Thus, there is also a problem to provide a dosage form which includes the paracetamol together with excipients useful to formulate the tablet into the dosage form and also excipients useful to ensure rapid disintegration, but not to provide a tablet that is too large for patient consumption or cannot be produced according to standard large scale manufacturing process. Suitably, this problem is typically magnified when paracetamol is combined with another pharmaceutically active agent (i.e. an NSAID) which may also be administered in a relatively high dose.

The present invention seeks to provide a process for the manufacture of a composition comprising both paracetamol and a NSAID which overcomes at least some of the aforementioned disadvantages. In particular, the present invention seeks to provide a composition capable of tabletting with minimum tabletting excipients and having advantageous tabletting, disintegration and dissolution properties.

We have now found that if a mixture comprising a molten NSAID and paracetamol contained therein is solidified and formed into granules, the granules typically exhibit improved flow characteristics, improved compressibility and may be formed directly into tablets which typically exhibit advantageous disintegration and dissolution properties is provided.

Thus, according to a first aspect the present invention provides a process for producing a granular composition comprising a plurality of solidified melt granules including a non-steroidal anti-inflammatory drug (NSAID) and paracetamol, the process comprising the steps of:
(a) forming a melt mixture by mixing a molten NSAID free acid and paracetamol, optionally with one or more excipients; and
(b) forming the melt mixture into solidified melt granules, wherein the paracetamol is present in an amount of greater than or equal to 40% by weight and less than or equal to 80% by weight of the solidified melt granules based on the total weight of the solidified melt granules.

Such a process may be referred to hereinafter as the process of the present invention.

Preferably, step (a) of the process of the present invention takes place in a melt extruder. Preferably, in step (b) the melt mixture is cooled to form a solidified melt and the solidified melt is formed into a plurality of solidified melt granules. The plurality of solidified melt granules may be formed by comminuting the solidified melt.

Unexpectedly, the granular composition obtainable by the process of the present invention typically exhibits improved flow characteristics and it is readily compressible, unlike untreated paracetamol. Suitably, the granular composition may be compressed directly into tablets with minimum tabletting excipients. Suitably, tablets formed from the granular composition typically exhibit increased hardness compared with softer tablets formed from a dry blend of paracetamol and ibuprofen. The tablets formed from the granular composition typically are sufficiently hard to withstand the further rigours of the manufacturing process i.e. film coating. Conveniently, the granular composition lends itself for formulation into solid dosage forms as it is easily compressible and tends not to stick to the punches of a tabletting machine. Suitably the throughput of a compression process employing the granular composition obtainable by the process of the present invention is substantially increased compared with employing a blend of untreated paracetamol and the NSAID. Furthermore, it is typically not necessary to pre-treat the paracetamol (i.e. employing a granulation process to improve its flowability) or employ direct compression forms of paracetamol in the process of the present invention, the paracetamol may be taken directly, without pre-treatment, from a bulk production process. Suitably, the process of the present invention may permit a reduction in the overall costs and provide a less complex manufacturing process for producing solid dosage forms containing a NSAID and paracetamol.

Further advantages of the granular composition obtainable by the process of the present invention lie in the relatively small amount of additional tabletting excipients needed to prepare a dosage form, in particular a solid dosage form for oral administration, thus allowing smaller dosage forms to be produced containing a relatively high concentration of NSAID and paracetamol, thereby increasing patient compliance.

Unexpectedly, it has been found that pharmaceutical compositions, in particular a solid dosage form for oral administration, prepared from the granular composition obtainable from the process of the present invention have valuable disintegrating properties. Moreover, the dissolution results of such pharmaceutical compositions typically exhibit an unexpectedly high level of the NSAID and paracetamol dissolved in the aqueous medium after relatively short periods of time.

Thus the process of the present invention typically provides advantages in processing NSAIDs and paracetamol, improved patient compliance, improved hardness of solid dosage forms with desirable disintegration and dissolution properties, and a lowering of the overall costs of tablets formed from NSAIDs and paracetamol.

In the process of the present invention the NSAID free acid is melted. Thus the terms "melt" and "molten", as used herein, mean the NSAID free acid must melt, either fully or at least partially, during formation of the granular composition. Preferably, the NSAID free acid is fully melted during the preparation of the granular composition.

By the term "NSAID free acid" we mean that the NSAID includes an acidic group which is not derivatised for example by the formation of a salt. Thus, the term "NSAID free acid" includes NSAIDs which include non-derivatised carboxylic acid groups *per se* (e.g. the propionic acids) and/or NSAIDs which include non-derivatised acidic enol groups (e.g. oxicams). It will be appreciated by those skilled in the art that a NSAID in the form of a free acid is physically and chemically distinct from the same NSAID in the form of a salt. Suitably, melt mixtures formed by melting NSAID salts only (i.e. by not melting any NSAID free acid) are not embraced by the process of the present invention. Preferably, the melt mixture is formed by melting NSAID free acids only. Suitably, the melt granules obtained by the process of the present invention are essentially free of NSAID salts.

The invention allows the formation of a granular component comprising a variety of NSAIDs, in particular NSAIDs which preferentially inhibit Cox-1.

Suitable types of NSAIDs which preferentially inhibit Cox-1 may be selected from the following categories:
(1) the propionic acids;
(2) the acetic acids;
(3) the fenamic acids;
(4) the biphenylcarboxylic acids;
(5) the oxicams.

Suitable propionic acids for use herein include ibuprofen, naproxen, benoxaprofen, flurbiprofen, fenoprofen, fenbufen, ketoprofen, indoprofen, pirprofen, carprofen, oxaprozin, prapoprofen, miroprofen, tioxaprofen, suprofen, alminoprofen, tiaprofenic acid, fluprofen, and bucloxic acid. Preferred members of the propionic acid group include ibuprofen, naproxen, flurbiprofen, fenoprofen, ketoprofen and fenbufen, especially ibuprofen.

Suitably acetic acids for use herein include indomethacin, sulindac, tolmetin, zomepirac, diclofenac, fenchlofenac, alchlofenac, ibufenac, isoxepac, furofenac, tiopinac, zidometacin, acemetacin, fentiazac, clidanac and oxipinac. Preferred members of the acetic acid group include tolmetin sodium, zomepinac sodium, sulindac and indomethacin.

The fenamic acids for use herein include mefenamic acid, meclofenamic acid, flufenamic acid, niflumic acid and tolfenamic acid. Preferred members of the fenamic acid group include mefenamic acid and meclofenamic acid.

The biphenylcarboxylic acids for use herein include diflunisal and flufenisal.

The oxicams for use herein include meloxicam, piroxicam, sudoxican, isoxicam. Preferred members of this group include piroxicam and meloxicam.

Suitably, the NSAIDs for use in the present invention typically exhibit isomerism. Suitably, all stereoisomers, diastereoisomers, enantiomers and mixtures therefore, including racemic mixtures, of the NSAIDs are embraced by the scope of the present invention.

A highly preferred class of NSAIDs for use in the process of the present invention are the propionic acids, particularly 2-aryl propionic acids, especially 2-phenyl propionic acids.

Highly preferred propionic acids include naproxen, flurbiprofen, ibuprofen and ketoprofen, particularly racemic mixtures and S(+)- enantiomers thereof. More preferred 2-aryl propionic acids include flurbiprofen and ibuprofen, particularly racemic mixtures and S(+)- enantiomers thereof. Even more preferred 2-aryl propionic acids include racemic flurbiprofen and racemic ibuprofen, especially racemic ibuprofen.

Preferred NSAIDs for use in the invention have relatively low melting points so that the melting process does not use significant amounts of energy, which thus reduces production costs. As stated above, a favoured class of compounds are the 2-arylpropionic acids which are generally substantially insoluble and have poor taste properties. Thus, typical melting points of the low melting NSAIDs fall within the range of 30 to 300°C. Preferred NSAIDs have melting points in the range 30-200°C (such as racemic naproxen, melting point 156°C), more preferably 30-160°C, further preferably 40-120°C (such as racemic flurbiprofen, melting point 114°C), most preferably 40-100°C (such as racemic ibuprofen (melting point 75-77°C), S(+)-ibuprofen (melting point 52-54°C) and racemic ketoprofen (melting point 96°C)). Preferred low-melting NSAIDs are naproxen, ketoprofen, flurbiprofen, ibuprofen. Preferably the NSAID is in the form of a racemic mixture or an enantiomer (especially the S(+)-enantiomers) thereof.

The invention is especially adapted for an ibuprofen medicament. The term "ibuprofen medicament" preferably includes racemic ibuprofen and S(+)-ibuprofen. Most advantageous results are obtained with racemic ibuprofen in its free acid form which has a high dosage combined with poor solubility properties.

Suitably, the granular composition and the melt granules may comprise one or more different NSAIDs as defined herein. Preferably, however, the granular composition and the melt granules comprise a single NSAID. Most preferably, the granular composition and the melt granules comprise a single NSAID in a single enantiomeric form or as a racemic mixture i.e. S(+)-ibuprofen only or racemic ibuprofen only. Moreover, as described hereinafter, the granular composition (i.e. the melt granules) or a pharmaceutical composition formed therefrom may include one or more further pharmaceutically active agents in addition to the NSAID and paracetamol. However, a highly preferred granular composition (i.e. the melt granules) or a pharmaceutical composition formed therefrom includes NSAIDs and paracetamol as the only pharmaceutically active agents, most preferably a single NSAID as defined herein and paracetamol.

Suitably, when the NSAID free acid is melted a liquid is formed. Paracetamol has a melting point of 169 to 172 °C and depending on the NSAID free acid employed and the process operating conditions, the paracetamol may partially or fully melt during formation of the granular composition. Preferably, the paracetamol essential does not melt during formation of the granular composition. Suitably, the paracetamol may partially dissolve within the molten NSAID free acid; however the majority of the paracetamol is typically dispersed within the molten NSAID free acid. Thus, the NSAID free acid melts and forms a melt phase having the paracetamol and other optional excipients contained therein. On cooling the molten NSAID free acid and paracetamol mixture, the NSAID typically forms a continuous crystalline type solid phase which enrobes or coats the paracetamol. The solidified melt may be milled directly into a granule that is suitable for compressing directly into a pharmaceutical dosage form with minimal tabletting excipients. In other words, the NSAID free acid at least in part loses its crystallinity and acts as a carrier for the paracetamol. Preferably, when the NSAID free acid is fully melted, the molten NSAID free acid on cooling forms a single continuous phase, namely a single continuous crystalline solid phase i.e. the crystalline structure of the NSAID is not interrupted by a NSAID having a different crystalline structure. This may occur, for example, if the NSAID is only partially melted where the crystalline structure of the melted NSAID is interrupted by the non-melted NSAID, thus providing that the NSAID does not have a single crystalline structure as the crystalline structure of the solidified melted NSAID is different (e.g. particle size) than the crystalline structure of unmelted NSAID.

Thus, as used herein, "solidified melt granules" means granules formed by mixing a molten NSAID free acid and paracetamol, optionally with one or more tabletting excipients, cooling to a temperature below the melting point of the NSAID and forming the solid mass into granules. The granular composition comprises a plurality of such granules.

Unexpectedly, if the NSAID free acid is fully melted during the preparation of the granular composition, then a resulting pharmaceutical composition (i.e. a solid dosage form) formed from the granular composition typically exhibits an improved dissolution and solubility profile in comparison to a comparable pharmaceutical composition formed by partial melting of the NSAID free acid. In this respect, a pharmaceutical composition formed from a granular composition where the NSAID free acid is fully melted typically releases a higher concentration of NSAID in an aqueous medium over a relatively short time compared with a comparable pharmaceutical composition formed from a granular composition where the NSAID free acid is partially melted.

Unexpectedly, it has been found that only the NSAID free acid needs to be melted in step (a) of the process of the present invention. The paracetamol is typically dispersed within the molten NSAID free acid but does not itself melt.

All of the benefits described above with respect to improved flow characteristics, processability and dissolution characteristics of the granular composition are typically maintained even though the paracetamol does not melt. Conveniently, the process of the present invention may be operated at a temperature so that the paracetamol does not melt, (i.e. at a temperature substantially less than the melting point of paracetamol) thereby reducing and/or preventing the possibility of the paracetamol degrading during formation of the granular composition.

Furthermore, and even more surprising, such benefits are typically still maintained even when the weight of paracetamol present in the melt granules is greater than the weight of ibuprofen present in the melt granules. In particular, it has been found that the melt mixture comprising the molten NSAID free acid, paracetamol and any other optional excipients still typically has the required fluidity so it may be processed and formed into melt granules by standard techniques (i.e. the melt mixture may be formed in an extruder and then allowed to flow out of the extruder onto a cooling belt as described hereinafter), despite the melt mixture containing a relatively small proportion of the melt phase (i.e. molten NSAID free acid). Unexpectedly, the melt granules containing a larger amount by weight of paracetamol than ibuprofen also typically exhibit substantially improved flow and compressibility characteristics compared with untreated paracetamol. It may therefore be compressed directly into tablets having desirable hardness with the minimum use of excipients. This is particularly surprising since it is known that paracetamol is a poorly compressible substance, yet a melt granule containing an excess of paracetamol exhibits substantially improved compressibility compared to paracetamol alone. Pharmaceutical compositions (i.e. tablets) formed from the melt granules containing an excess of paracetamol also exhibit desirable dissolution rates whereby a high level of NSAID and paracetamol are dissolved in an aqueous, particularly aqueous acidic, medium after relatively short periods of time. Suitably, such pharmaceutical compositions may deliver higher levels of NSAID and paracetamol in the acidic conditions of the gastrointestinal tract or stomach when swallowed by a patient in need thereof.

Conveniently, this may increase the on-set of therapeutic action of paracetamol and the NSAID. Although only theory, it is believed that the improved dissolution rate resides in the physical structure of the melt granule.

As the paracetamol is in excess, the solidified melt granules comprise particles of paracetamol partially coated and adhered to one another by the NSAID free acid. Paracetamol is readily soluble, in acidic aqueous conditions, whereas NSAID free acids are not. Thus, in aqueous conditions, the particles of paracetamol begin to dissolve and the thin layers of NSAID between these particles fall part and dissolution of these too occurs much more rapidly.

Hence, a further advantage of such a granular composition is that it is readily soluble.

Suitably, such characteristics are highly desirable in ibuprofen and paracetamol combination therapy as the paracetamol is typically administered in relatively high doses (i.e. 500 mg per unit dose) compared with the NSAID (i.e. 200 mg per unit dose).

Thus, according to a preferred aspect of the present invention, the weight of paracetamol in the solidified melt granules is greater than or equal to the weight of NSAID in the solidified melt granules, based on the total weight of the melt granules. More preferably, the weight of paracetamol in the solidified melt granules is greater than the weight of the NSAID in the solidified melt granules, based on the total weight of the melt granules.

Suitably, the weight of paracetamol in the granular composition is greater than or equal to, preferably greater than, the weight of the NSAID in the granular composition, based on the total weight of the granular composition.

Preferably, the ratio by weight of paracetamol to NSAID in the solidified melt granules is greater than 1:1, more preferably greater than or equal to 1.5:1, even more preferably greater than or equal to 2:1. Preferably, the ratio by weight of paracetamol to NSAID in the solidified melt granules is less than or equal to 10:1, more preferably less than or equal to 5:1, even more preferably less than or equal to 3:1.

An especially preferred ratio by weight of paracetamol to NSAID in the solidified melt granules is between 2.2:1 and 2.8:1, particularly approximately 2.5:1.

Preferably, the ratio by weight of paracetamol to NSAID in the granular composition is greater than 1:1, more preferably greater than or equal to 1.5:1, even more preferably greater than or equal to 2:1. Preferably, the ratio by weight of paracetamol to NSAID in the granular composition is less than or equal to 10:1, more preferably less than or equal to 5:1, even more preferably less than or equal to 3:1.

An especially preferred ratio by weight of paracetamol to NSAID in the granular composition is between 2.2:1 and 2.8:1, particularly approximately 2.5:1,

The paracetamol is present in an amount of greater than or equal to 40% by weight, more preferably greater than or equal to 45% by weight, even more preferably greater than or equal to 50% by weight, even more preferably greater than or equal to 55% by weight of the solidified melt granules based on the total weight of the solidified melt granules and in an amount of less than or equal to 80% by weight, more preferably less than or equal to 75% by weight, most preferably less than or equal to 70% by weight of the solidified melt granules based on the total weight of the solidified melt granules. Preferably, the paracetamol is present in an amount of greater than or equal to 40% by weight, more preferably greater than or equal to 45% by weight, even more preferably greater than or equal to 50% by weight, even more preferably greater than or equal to 55% by weight of the granular composition based on the total weight of the granular composition.

Preferably, the paracetamol is present in an amount of less than or equal to 80% by weight, more preferably less than or equal to 75% by weight, most preferably less than or equal to 70% by weight of the granular composition based on the total weight of the granular composition.

Preferably, the NSAID is present in an amount of less than or equal to 45% by weight, more preferably less than or equal to 40% by weight of the granular composition based on the total weight of the granular composition.

Preferably, the NSAID is present in an amount of greater than or equal to 20% by weight, more preferably greater than or equal to 25% by weight of the granular composition based on the total weight of the granular composition.

Preferably, the NSAID is present in an amount of less than or equal to 45% by weight, more preferably less than or equal to 40% by weight of the solidified melt granules based on the total weight of the solidified melt granules.

Preferably, the NSAID is present in an amount of greater than or equal to 20% by weight, more preferably greater than or equal to 25% by weight of the solidified melt granules based on the total weight of the solidified melt granules.

Preferably, the paracetamol and any other optional tabletting excipients are uniformly dispersed within the molten NSAID free acid. A uniform mixture is thus produced. The mixture is allowed to cool by methods hereinafter discussed until a solid is produced. As the mixture cools, it becomes more viscous. The solidified mixture is then formed into granules.

In the process of the present invention, the NSAID free acid, paracetamol and other optional excipients to be included in the melt granules may be mixed in the solid state prior to melting the NSAID free acid. Alternatively, the paracetamol and/or other optional excipients to be included in the melt granules may be added to the molten NSAID free acid. Processes in which one or more additional components are mixed with the NSAID free acid and paracetamol prior to melting the NSAID free acid and a process in which one or more additional components are added to a melt mixture comprising the molten NSAID free acid and paracetamol are also within the scope of the present invention. An especially preferred method involves combining in the solid state the NSAID free acid, paracetamol and any optional additional excipients to be included in the melt granule, and then melting the NSAID free acid. Although the melting process may be carried out in a number of ways, for example heating the NSAID free acid and paracetamol in a suitable vessel, preferably the melting process is carried out by a melt extrusion process as detailed hereinafter.

Suitably, the melt mixture may be formed by melting the NSAID free acid and then adding the paracetamol and optionally one or more expicients to the molten NSAID free acid. Preferably, the melt mixture is formed by mixing the NSAID free acid, paracetamol and one or more optional excipients in the dry state and then melting the NSAID free acid.

The melt mixture is allowed to solidify in any manner found convenient. This includes both rapid cooling and slow cooling. Preferably, the melt mixture is cooled rapidly (i.e. quenched) as described herein. Typically, this allows the molten NSAID to form a single continuous crystalline phase. For example, the melt mixture may be allowed to cool in a cooled vessel. The melt mixture may be poured onto cooling trays which may be static or continuously moving. Static trays may be placed in cooling cabinets. Moving trays or belts may have additional cooling means, such as cooled water. The cooled melt mixture forms a solid and may be scraped off the belt or collected as it falls off one end of a continuously moving belt.

The solidified melt of the NSAID and paracetamol may be formed into granules by a plurality of methods. For example, it may be pulverised into granules. It may be milled and/or sieved. It may also be passed through a spray device such as a spray tower or spray granulator in which the molten material is sprayed from an orifice into a stream of cooled air, allowed to congeal/solidify and then collected. If the melt is extruded, the extrudate maybe cooled and then broken into conveniently sized pieces, followed by milling and or sieving. Alternatively, the extrudate may be extruded through holes and chopped into suitably sized granules for tabletting.

According to a preferred aspect, the granular composition is formed by a melt extrusion process. Such apparatus is commonly used and is familiar to one skilled in the art. A suitable extruder for use in the present invention is disclosed in WO 02/098391 and is available from APV, UK, Ltd model number APV MPC40.

In the first step of the process, a composition comprising the NSAID free acid, paracetamol and any other optional excipients are fed into the barrel of a heated extruder. At least the NSAID free acid is melted in the extruder thereby forming a melt mixture comprising of molten NSAID free acid, paracetamol and other optional excipients. Under conditions of pressure, the NSAID free acid may be melted at a temperature below its normal melting point. The maximum temperature is determined by the stability of the molten NSAID, the paracetamol and any further ingredients combined therewith. The NSAID free acid may be heated to any convenient temperature. Generally, the higher the temperature, the more quickly the NSAID free acid will melt although this must be balanced by the energy input required to heat the NSAID free acid. For highest efficiency, it is generally envisaged that the NSAID free acid will be heated to not more than 50°C, preferably 1-25°C and more preferably 5-20°C, above its melting point to keep energy costs to a minimum. A preferred heating range is 30-160°C, more preferably 35-140°C and most preferably 40-120°C.

In preferred embodiments according to the present invention, the NSAID free acid component is melted and the paracetamol remains in solid form. Thus, the composition is preferably heated at a temperature greater than the melting point of the NSAID free acid but less than the melting point of paracetamol.

The NSAID free acid is generally melted in a heated extruder barrel having an inlet for the solid composition and an outlet for the molten extrudate. By molten extrudate is meant the composition in which the NSAID free acid is molten, preferably fully molten, along with selected excipients, when present. Other excipients, and the paracetamol, may not, in fact, be molten themselves but are carried with the molten NSAID through the extruder and are dispersed within it. The barrel may be divided into different heating zones as desired. In addition, the work on the NSAID by the screw configuration in the extruder will also contribute to melting the NSAID free acid, thereby reducing its external applied temperature requirement. Accordingly the extruder barrel may be heated to a temperature less than the melting point of the NSAID free acid. For example, the normal melting point of racemic ibuprofen is 75-77°C, however under conditions of force/pressure (such as may be encountered in an extruder or similar processing device), the external applied heat necessary to melt the ibuprofen may be reduced significantly through the mechanical heat generated by the intense mixing action within the extruder. It is generally envisaged that the extruder will be heated to a temperature not less than 25°C below the melting point of the NSAID free acid, preferably in the range from 20°C below the melting point of the NSAID free acid to 50°C above the melting point of the NSAID free acid, more preferably from 15°C below the melting point of the NSAID free acid to 25°C above its melting point and most preferably to a temperature in the range of 10°C on each side of the melting point of the NSAID free acid. Some extruders allow different zones to be heated to different temperatures in the extruder. These temperatures can be chosen as desired to ensure that the NSAID free acid is fully melted in the first step (a) of the process of the present invention. Preferably, the composition is heated to a temperature in the range 80-130°C, more preferably 100-120°C to melt the NSAID free acid. When the NSAID free acid is ibuprofen it may conveniently be heated in the range 50-130°C, more preferably 60-100°C. The temperature of the ibuprofen in the extruder barrel is preferably in the range 66-96°C, preferably 70-82°C.

The extruder may also have one or more cooling zones. The cooling zones may be necessary to remove the heat generated by the kneading action on the material being extruded, particularly to ensure that there is a good flow of material into the extruder and out from the extruder.

In a preferred process according to the present invention, the extruder is provided with a cooling zone and a heating zone. More preferably, there is provided a cooling zone at the inlet portion of the extruder so that the material entering the extruder may be conveyed or transferred along the extruder to a heated zone. In the cooling zone, the internal heat generated within the material being extruded is carried away so that partial melting of the composition cannot occur which may be detrimental to the throughput of material in the extruder. Preferably, the extruder is provided with a cooled transfer zone and a heated melting zone.

In an especially preferred process, there is provided a heated zone at an end portion of the extruder at or adjacent the outlet. The extruded material may be heated to ensure that the extrudate passing through the extruder outlet is sufficiently heated so that the temperature difference between the molten extrudate and extrudate cooling means is maximised as appropriate to optimise the cooling process. For example, the barrel may be heated to cause the NSAID free acid component of the extrudate passing through the outlet to be preferably fully molten or substantially fully molten. The pressure within the extruder may cause a lowering of the melting point of the NSAID free acid. Accordingly, preferably, the temperature of the extrudate passing through the outlet is in the range of 20°C on each side of the normal melting point of the NSAID free acid, preferably within 10°C on each side of the melting point of the NSAID free acid.

The extruder is suitably provided with at least one screw shaft provided with means arranged to generate heat within the composition. This may usually be achieved by a combination of kneading paddles and helical screws. Generally, it is preferred to provide helical screws at the inlet portion to convey the material away from the inlet. The material may be extruded in the extruder barrel with screws and/or with paddles. It is preferred to use more than one screw shaft, for example a twin-screw shaft, to maximise the extrusion effect on the material being extruded. The use of paddles also maximises the shear effect on the material being extruded. The paddles may be offset at any desired angle or combination of angles to generate internal heat within the composition as appropriate to melt the NSAID component. The configuration and/or size of the paddles will depend on factors such as the diameter and/or length of the extruder, the ratio of the length to the diameter, the extruder speed, the torque applied and the desired temperature to melt the NSAID. The screws and/or the paddles may be in the forward and/or reverse direction to maximise the pressure within the mixing zone as desired.

A preferred arrangement comprises helical transfer screws at the inlet portion of the extruder, a plurality of paddles which may have differing sizes and degrees to which they are offset and further helical transfer screws at the outlet portion to convey the extrudate out of the extruder. Further preferably the helical transfer screws at the outlet portion may comprise a reverse helix followed by a forward helix.

In the process of the present invention, the second step requires the cooling of the extruded composition.

Preferably, the melt is cooled rapidly (i.e. quenched). Material cooled in such a way typically comprises an NSAID phase in which paracetamol particles are dispersed. The NSAID typically forms a continuous crystalline phase which coats the paracetamol particles.

Preferably the extrudate is formed into two or more thin ribbons. This is preferably achieved by passing the molten extrudate through channels at the outlet which form streams or ribbons of extrudate which may be directed onto the cooling means, preferably a cooling belt or a cooling drum.

The ribbons of molten extrudate are cooled rapidly by said cooling means, i.e. the ribbons solidify in 5 minutes or less, preferably in 3 minutes or less, more preferably in 1 minute or less (e.g. 0-60 seconds), preferably in 50 seconds or less (e.g. 1-50 seconds), more preferably 1-40 seconds and most preferably 1-30 seconds.

Suitably, the width of each ribbon of molten extrudate is greater than the depth of the ribbon so that cooling is optimised. The width of each ribbon will depend, at least to some extent, on the viscosity of the molten extrudate. Preferably, each ribbon of molten extrudate has a depth on the cooling means of 10 mm or less, preferably up to 6 mm (for example 0.1-6 mm), preferably 0.5-5 mm, for example 3-4 mm and most preferably 1-3 mm, for example 2 mm.

Cooling will normally occur first on the side of the ribbon proximate the cooling means. Accordingly, usually the lower surface of the ribbons solidifies while the upper surface of the ribbon is still molten. As the ribbon is further cooled, the extrudate solidifies throughout its depth.

To maximise output, a plurality of ribbons are provided extending parallel to each other, for example on a cooling belt. Preferably, there are more than two ribbons, for example three, four, five, six, seven, eight, nine or ten or more ribbons according to the size of the extruder. The number of ribbons may be limited by the width of the ribbon formed and the whole width of the cooling means which provides for a maximum number of ribbons. It has been found that the ribbons of molten extrudate do not spread on the cooling means, accordingly there requires only a small space between the ribbons.

To achieve rapid cooling, it is preferable to have a significant temperature difference between the molten extrudate and the cooling means as the extrudate comes into contact with the cooling means, for example at least 25°C, preferably at least 35°C, more preferably at least 45°C and most preferably at least 55°C. The upper end of the above ranges is limited by the melting point of the NSAID, but it is not desired to heat the extruded material to too high a temperature as the extra energy costs will not be balanced by any processing advantage. Accordingly, a practical upper limit to each of the above ranges is 100°C, more usually 80°C.

Generally, it is expected that the molten extrudate will be cooled to a temperature below the melting point of the NSAID free acid before being formed into granules. The molten composition may be poured onto cooling trays which may be static or continuously moving. Static trays may be placed in cooling cabinets. Moving trays or belts may have additional cooling means, such as cooled water. The cooled melt forms a solid and may be scraped off the belt or collected as it falls off one end of a continuously moving belt. Preferably the molten composition may be cooled by passing the molten mixture onto a moving cooling belt, preferably a continuously rotating cooling belt. Preferably, the belt is cooled by water. The water may be applied to the underside of the belt along its length or partially along its length as desired and according to the length of the belt, the quantity of molten composition and the speed of the belt. It is especially preferred to cool the molten composition at least initially by cooling means, for example until the NSAID component has started to solidify. Advantageously, the belt is water-cooled along substantially the whole of its length and it is of minimum length required (e.g. 3-7m) to allow the NSAID to cool to the solid state.

As mentioned above, the cooled composition typically comprises particles of paracetamol dispersed within a matrix of NSAID material.

The solidified melt may be formed into granules by a plurality of methods. For example, it may be pulverised or comminuted into granules. It may be milled and/or sieved. If it is cooled on a moving belt or drum, the cooled melt may be broken into conveniently sized pieces, followed by milling and or sieving.

The granular composition may be sieved to ensure that the melt granules are of the appropriate size for efficient tabletting. The granules produced on cooling the molten composition are preferably of a suitable size for tabletting, preferably in a standard large scale tabletting machine. The melt granules in the granular composition preferably have a mean particle size in the range 10-2000µm, more preferably 50-1000µm and most preferably 100-400µm.

Valuable results are achieved when the bulk density of the melt granules is in the range 0.1-1gml⁻¹, more preferably 0.3-0.6gml⁻¹. Further preferred properties are obtained when the tapped density is in the range 0.3-0.7gml⁻¹ (more preferably 0.4-0.6 gml⁻¹). Further, preferably the melt granules have a porosity of 0.5-2.0 g/ml.

According to a second aspect of the invention, there is provided a granular composition comprising a plurality of solidified melt granules comprising a NSAID free acid as defined herein, paracetamol and optionally one or more excipients as defined herein, wherein the paracetamol is present in an amount of greater than or equal to 40% by weight and less than or equal to 80% by weight of the solidified melt granules based on the total weight of the solidified melt granules.

Preferably, essentially all of the NSAID in the melt granules is in the form of the free acid. Preferably, the paracetamol is in particulate form dispersed within a solidified melt of the NSAID free acid.

Preferably, the weight of paracetamol in the melt granules is greater than or equal to, more preferably greater than, the weight of NSAID free acid in the melt granules, based on the total weight of the melt granules. The highly preferred ratio by weight of paracetamol to NSAID, percent by weight of NSAID and percent by weight of paracetamol which may be present in the melt granules and/or the granular composition has been defined hereinbefore.

According to a third aspect, the present invention provides a granular composition comprising a plurality of solidified melt granules comprising a NSAID as defined herein and paracetamol, and optionally one or more excipients as defined herein, wherein the granular composition is obtainable by the process according to the first aspect of the present invention.

Preferably, the granular composition according to the second aspect of the present invention or obtainable by the process of the first aspect of the present invention consists of the melt granules only. The melt granules may consist of the NSAID and paracetamol only. Alternatively, the melt granules may further include one or more expicients as defined herein. However, it will be appreciated that a prime advantage of the present invention is that the number of excipients necessary to achieve a quickly disintegrating dosage form, such as a compressed tablet, with good dissolution characteristics is minimal.

A highly preferred excipient for inclusion in the melt granules is a disintegrating agent. Preferably, the solidified melt granules of the granular composition includes one or more disintegrating agents.

A disintegrating agent may also or alternatively be present as an extra-granular component used in tablet formation. Preferably, the disintegrating agent is present within the granular composition, even more preferably the disintegrating agent is only present within the granular composition. If a disintegrating agent is incorporated into the melt mixture, comprising the molten NSAID and paracetamol, and intimately combined therewith, the mixture cooled and milled to produce a granule, a pharmaceutical composition capable of tabletting with minimum tabletting excipients and having advantageous tabletting, disintegration and dissolution properties is provided. The disintegrating agent has the effect of causing a solid dosage form, such as a tablet, formed from the granular composition to disintegrate quickly under the conditions found in the gastro-intestinal tract. Examples of disintegrating agents include one or more of wheat starch, maize starch, potato starch, sodium starch glycolate, low-substituted hydroxypropyl cellulose, alginic acid, cross-linked polyvinylpyrrolidone, magnesium aluminium silicate and croscarmellose sodium. Preferred disintegrating agents are those which swell on the action of water thus causing the ingredients in the granular composition, and solid dosage forms prepared therefrom, to be pushed apart and out into the aqueous disintegration medium. Preferred disintegrating agents comprise one or more of croscarmellose sodium and sodium starch glycolate, especially croscarmellose sodium.

Preferably, the disintegrating agent is present in an amount of less than or equal to 20% by wt, more preferably less than or equal to 15% by wt, even more preferably less than or equal to 10% by wt of the granular composition. Preferably the disintegrating agent is present in an amount of greater than or equal to 1% by wt, more preferably greater than or equal to 2% by wt, most preferably greater than or equal to 3% by wt of the granular composition.

Preferably, the disintegrating agent is present in an amount of less than or equal to 20% by wt, more preferably less than or equal to 15% by wt, even more preferably less than or equal to 10% by wt of the solidified melt granules.

Preferably the disintegrating agent is present in an amount of greater than or equal to 1% by wt, more preferably greater than or equal to 2% by wt, most preferably greater than or equal to 3% by wt of the solidified melt granules.

Preferably, the percent by weight ratio of NSAID to disintegrating agent in the solidified melt granules is 20:1 to 2:1, more preferably 15:1 to 5:1, most preferably 12:1 to 7:1.

Preferably, the percent by weight ratio of paracetamol to disintegrating agent in the solidified melt granules is 20:1 to 2:1, more preferably 15:1 to 5:1, most preferably 12:1 to 7:1.

Optionally, the granular composition, and the solidified melt granules, may further comprise additional excipients such as a diluent or a surfactant. Preferably, the granular composition, and the solidified melt granules, contains only a NSAID, paracetamol and a disintegrating agent.

It will be appreciated that the granular composition may be prepared in accordance with the present invention by a simple cost-efficient manufacturing process on a large scale. Formulations prepared from the granular composition according to the present invention have been found to be stable on storage and to have advantageous dissolution properties. The granular composition typically exhibits desirable flow and compressibility characteristics and it may be tabletted using minimum excipients without sticking or capping during the tabletting process to provide a dosage form having suitable hardness properties combined with advantageous disintegration properties. Furthermore, the poor taste associated with certain NSAIDs is significantly improved.

The solidified melt granules may be formulated directly or they may be combined with an extra-granular component and formulated into a unit dosage form.

Thus according to a fourth aspect, the present invention provides a pharmaceutical composition in the form of a unit dosage for oral administration comprising a granular composition, as defined herein, including a plurality of solidified melt granules comprising a NSAID free acid, paracetamol and optionally one or more excipients. Preferably, the unit dosage form is a solid unit dosage form.

The unit dosage form may be swallowed, dispersed in water prior to ingestion or adapted to disintegrate in the mouth. Preferably, the unit dosage form is adapted to release the NSAID and paracetamol in the stomach or the gastro-intestinal tract. Most preferably, the unit dosage form is swallowed by a patient in need thereof.

Suitable unit dosage forms include compressed tablets, chewable tablets, effervescent formulations, trouches. Most preferably, the unit dosage form is in the form of a compressed tablet, especially a non-effervescent compressed tablet.

The compressed tablet may optionally be coated with a film coat, for example based on a conventional cellulose polymer such as hydroxypropylmethyl cellulose, or a conventional sugar coat, for example base on sucrose or lactose.

The proportion of NSAID in the unit dosage form will depend on the dose required for the desired therapeutic effect. Low dose drugs such as flurbiprofen and ketoprofen may be present in an amount of as little as 10% by weight of the unit dosage form. However, a preferred feature of the invention is that high dose NSAIDs, such as ibuprofen, can be formulated in combination with paracetamol into smaller dosage forms than comparable dosage forms produced by standard tabletting techniques (e.g. dry blending the component parts and subsequently compressing the mixture).

Preferably, the NSAID is present in an amount of greater than or equal to 10% by weight, more preferably greater than or equal to 15% by weight, even more preferably greater than or equal to 20% by weight of the unit dosage form based on the total weight of the unit dosage form.

Preferably, the NSAID is present in an amount of less than or equal to 45% by weight, more preferably less than or equal to 40% by weight, even more preferably less than or equal to 35% by weight, most preferably less than or equal to 30% by weight of the unit dosage form based on the total weight of the unit dosage form.

Preferably, the paracetamol is present in an amount of greater than or equal to 35% by weight, more preferably greater than or equal to 40% by weight, even more preferably greater than or equal to 45% by weight, even more preferably greater than or equal to 50% by weight, most preferably greater than or equal to 55% by weight of the unit dosage form based on the total weight of the unit dosage form.

Preferably, the paracetamol is present in an amount of less than or equal to 80% by weight, more preferably less than or equal to 75% by weight, even more preferably less than or equal to 70% by weight, even more preferably less than or equal to 65% by weight, most preferably less than or equal to 57% by weight of the unit dosage form based on the total weight of the unit dosage form.

Preferably, the NSAID and paracetamol, and disintegrating agent when present, are only present within the granular composition of the unit dosage form. The ratios by weight of NSAID to paracetamol, NSAID to disintegrating agent, paracetamol to disintegrating agent, and the amount of disintegrating agent present as stated in respect of the granular composition apply equally and independently to the unit dosage form.

Unit dosages for effective therapy are known to those skilled in the art for each NSAID. For example, they may comprise the NSAID to an extent of 5 mg, 10 mg, 12.5 mg, 25 mg, 50 mg, 100 mg, 150 mg, 200 mg, 250 mg, 300 mg, 350 mg, 400 mg, 500 mg, 600 mg and 800 mg. Where derivatives are employed, normally the precise unit dosages are chosen to give the equivalent NSAID doses given above. For the treatments described herein the maximum daily dose of ibuprofen is generally 3200 mg. A single unit daily dose may be 100 mg. Preferred unit doses are in the range 100-400 mg, more preferably 100-300 mg and especially 200 mg ibuprofen. The maximum daily dose of flurbiprofen is generally 300 mg. A single unit dose may be 12.5 mg. Preferred unit doses are in the range 12.5-150 mg, more preferably 25-100 mg and especially 50 mg flurbiprofen. The maximum daily dose of naproxen is generally 1500 mg. A single unit daily dose may be 125 mg. Preferred unit doses are in the range 220-750 mg, more preferably 220-500 mg and especially 220-250 mg naproxen. The maximum daily dose of ketoprofen is generally 200 mg. A single unit daily dose may be 25 mg. Preferred unit doses are in the range 25-100 mg, more preferably 25-75 mg and especially 50 mg ketoprofen.

Unit dosages for effective therapy are known to those skilled in the art for paracetamol. For example, they may comprise 100 mg, 150 mg, 200 mg, 250 mg, 300 mg, 350 mg, 400 mg, 450 mg, 500 mg, 550 mg, 600 mg, 650 mg, 700 mg, and 800 mg. Preferred unit doses are in the range of 100 to 650 mg, more preferably 200 to 550 mg, most preferably 250 to 500 mg.

Preferred unit dosage forms comprise of 500 mg of paracetamol and 200 mg of ibuprofen, 325 mg of paracetamol and 200 mg of ibuprofen, and 1000 mg of paracetamol and 400 mg of ibuprofen.

Preferably, the granular composition as defined herein is combined with an extra-granular component and then formulated into a unit dosage form. The extra-granular component comprises the ingredients incorporated in the unit dosage form which are not contained in the solidified melt granules. They may be mixed with the solidified melt granules simultaneously or at sequential stages in the process to prepare unit dosages. A particular advantage of the present invention is that all of the ingredients of the extra-granular component may be combined with the granular composition at the same time and also there typically does not have to be significant processing of the ingredients in the extra-granular component prior to combining with the granular composition. The melt granules may be combined with the extra-granular component by conventional mixing and blending techniques so as to form a uniform mixture of ingredients. Examples of apparatus which may be used to facilitate this process are: Ribbon Blender, IBC Blender, V-Blender and Plough Blender. The uniform mixture is typically a dry blend which may be compressed into tablets using standard tabletting machines.

Suitably, a preferred unit dosage form comprises:
(a) a granular composition, as defined herein, comprising a plurality of solidified melt granules comprising a NSAID free acid, paracetamol and optionally one or more excipients; and,
(b) an extra-granular component comprising one or more ingredients as defined herein.

As stated previously the most preferred unit dosage form is a non-effervescent compressed tablet.

Preferably, the unit dosage form comprises 60 to 99.5% by weight, more preferably 75 to 95% by weight, even more preferably 80 to 90% by weight of the granular composition based on the total weight of the unit dosage form.

Preferably, the unit dosage form comprises 0.5 to 40% by weight, more preferably 5 to 25% by weight, even more preferably 10 to 20% by weight of the extra-granular component based on the total weight of the unit dosage form.

Suitable ingredients which may be present in the extra-granular component, or may be employed as excipients in the granular composition, include any of the following:

### A water-soluble or water-insoluble diluent

Suitable water-soluble diluent materials include sugars (such as sucrose, fructose, lactose, dextrose), cyclodextrin, maltodextrin and salts of organic acids (e.g. sodium citrate and potassium citrate).

Suitable water-insoluble diluent materials include cellulose derivatives (such as microcrystalline cellulose) starch and derivatives thereof (such as pre-gelatinised starch), dicalcium phosphate, tricalcium phosphate, calcium sulphate, calcium carbonate. Microcrystalline cellulose and dicalcium phosphate are preferred water insoluble diluents.

If present, the diluent may be present in an amount of 0.1 to 25% by weight, more preferably 0.1 to 20% by weight, even more preferably 1 to 15% by weight, most preferably 4 to 15% by weight of the unit dosage form based on the total weight of the unit dosage form.

Although the diluent may be present in the melt granules and/or the extra-granular component, preferably if the diluent is present it is exclusively within the extra-granular component of the unit dosage form.

### Surfactant

Preferred surfactants are sodium lauryl sulphate and poloxamer. The surfactant may be used to an extent of 0.05 to 8% by weight, more preferably 0.1 to 5% by weight, most preferably 0.2 to 2% by weight of the unit dosage form based on the total weight of the unit dosage form. Although the surfactant may be present within the melt granules, preferably if a surfactant is present in the unit dosage form it is present exclusively within the extra-granular component.

### A "wicking agent"

The term "wicking agent" refers to any excipient that forms capillary pathways within a compact, such as a tablet, such that when the compact is placed in an aqueous environment liquid is drawn through the pathways by capillary action, disintegration of the compact occurs as interparticulate bonds are ruptured by the ingress of liquid. The wicking agent is insoluble in water and is preferably present only in an extra-granular composition used in tabletting.

The insoluble wicking agent is selected from inorganic materials, starch materials, cellulose materials such as hydroxyethylcellulose (HEC), hydroxypropylcellulose (HPC), hydroxypropylmethyl cellulose (HPMC), and mixtures thereof. Preferably the inorganic material comprises silicon dioxide, PTFE powder, alkali metal silicates, alkaline earth metal silicates, alkali metal carbonates and bicarbonates and alkaline earth metal carbonates. Examples include sodium carbonate, sodium bicarbonate, potassium carbonate, magnesium carbonate, calcium carbonate, PTFE powder, sodium silicate, potassium silicate, magnesium silicate and calcium silicate. Preferably the starch material comprises starches such as potato starch, maize starch, rice starch, tapioca starch and starch derivatives including modified starches such as pre-gelatinised starch. More preferably, the wicking agent comprises at least one of silicon dioxide and/or alkaline earth metal carbonates, especially calcium carbonate, talc, maize starch and pre-gelatinised starch. Most preferably, the wicking agent comprises silicon dioxide.

Preferably, the wicking agent especially silicon dioxide is present in an amount of 0.05 to 10% by weight, more preferably 0.05 to 5% by weight, even more preferably 0.1 to 3% by weight, most preferably 0.2 to 1% by weight of the unit dosage form based on the total weight of the unit dosage form.

Most preferably, the wicking agent is present exclusively within the extra-granular component.

### A lubricant

Conventional lubricants for ibuprofen tablets may be used for example stearic acid, sodium lauryl sulphate, polyethylene glycol, hydrogenated vegetable oil, sodium stearyl fumarate, magnesium stearate or calcium stearate. These may be incorporated in the unit dosage form in amounts of 0.05 to 5% by weight, preferably 0.1 to 3% by weight based on the total weight of the unit dosage form. Although the lubricant, if present in the unit dosage form, may be incorporated in the solidified melt granules and/or the extra-granular component, preferably the lubricant is only present within the extra-granular component.

### A further pharmacologically active ingredient and/or enhancing agent

Suitable agents may include any other ingredient commonly used in a composition useful to treat pain, inflammation and/or fever, for example caffeine or another xanthine derivative, another analgesic, for example codeine, a skeletal muscle relaxant: an antihistamine (e.g. acrivastine, astemizole, azatadine, azelastine, bromodiphenhydramine, brompheniramine, carbinoxamine, cetirizine, chlorpheniramine, cyproheptadine, dexbromopheniramine, dexchloropheniramine, diphenhydramine, ebastine, ketotifen, lodoxamide, loratidine, levocabastine, mequitazine, oxatomide, phenindamine, phenyltoloxamine, pyrilamine, setastine, tazifylline, temelastine, terfenidine, tripelennamine or triprolidine (preferably non-sedating antihistamines are employed)); a decongestant (e.g. pseudoephedrine, phenylpropanolamine and phenylephrine); a cough suppressant (e.g. caramiphen, codeine or dextromethorpan); an expectorant (e.g. guaifenesin, potassium citrate, potassium guaiacolsuphonate, potassium sulphate and terpin hydrate); an anti-ulcer histamine antagonist (e.g. misoprostol); and/or an anti-nausea drug (e.g. domperidone). The further pharmacologically active ingredient and/or enhancing agent may be incorporated in the melt granules and/or the extra-granular component.

Other conventional tabletting excipients known to the person skilled in the art may be incorporated in the granular composition according to the present invention as desired, although it will be appreciated that a prime advantage of the present invention is that the number of excipients necessary to achieve a quickly disintegrating dosage form, such as a compressed tablet, with good dissolution characteristics is minimal.

A preferred unit dosage form, especially a compressed tablet, particularly a non-effervescent compressed tablet comprises:
a) a granular composition as defined herein comprising a plurality of solidified melt granules of a NSAID and paracetamol uniformly contained therein; and
b) an extra-granular component comprising an insoluble wicking agent present in an amount of 0.05 to 5.0% by weight based on the total weight of the unit dosage form.

A further preferred unit dosage form, especially a compressed tablet composition, particularly a non-effervescent compressed tablet comprises:
a) a granular composition comprising a plurality of solidified melt granules of a NSAID, paracetamol and a disintegrant uniformly contained therein; and
b) an extra-granular component comprising an insoluble wicking agent present in an amount of 0.05 to 5.0% by weight based on the total weight of the unit dosage form.

Preferably, the insoluble wicking agent comprises silicon dioxide.

A still further preferred unit dosage form, especially a compressed tablet, particularly a non-effervescent compressed tablet comprises:
(a) a granular composition comprising a plurality of solidified melt granules of ibuprofen, paracetamol and a disintegrating agent; and
(b) an extra-granular component comprising an insoluble wicking agent present in an amount of 0.05 to 5% by weight, based on the total weight of the unit dosage form.

Preferably, the extra-granular component further includes a diluent present an amount of 4 to 15% by weight based on the total weight of the unit dosage form.

Preferably, the granular composition as defined hereinbefore is present in an amount of 60 to 99.5% by weight, more preferably 75 to 95% by weight, even more preferably 80 to 90% by weight of the unit dosage form based on the total weight of the unit dosage form.

Preferably, the extra-granular component as defined hereinbefore is present in an amount of 0.5 to 40% by weight, more preferably 5 to 25% by weight, most preferably 10 to 20% by weight of the unit dosage form based on the total weight of the unit dosage form.

It will be appreciated that the ibuprofen, paracetamol and the disintegrating agent may be present in the amounts as defined hereinbefore.

Suitably, the present invention extends to a process of forming a compressed dosage form, particularly a non-effervescent compressed dosage form, comprising compressing said granular composition as defined herein, optionally with an extra-granular component as defined herein, to form a compressed dosage form.

Preferably, the compressed dosage form comprises a non-effervescent compressed dosage form, especially a non-effervescent compressed tablet.

NSAIDs are primarily anti-inflammatory, analgesic and anti-pyretic agents but have also been proposed for other therapeutic uses, including the treatment of periodontal bone loss, pruritus and Alzheimer's disease. Paracetamol possesses analgesic and anti-pyretic activity. The unit dosage forms of the present invention are therefore indicated for use in the treatment of all therapeutic uses for which cyclooxygenase inhibitors are effective, including rheumatoid arthritis, osteoarthritis, ankylosing spondylitis, seronegative arthropathies, periarticular disorders and soft tissue injuries. They may also be used in the treatment of postoperative pain, postpartum pain, dental pain, dysmenorrhoea, headache, migraine, rheumatic pain, muscular pain, backache, neuralgia and/or musculoskeletal pain or the pain or discomfort associated with the following: respiratory infections, colds or influenza, gout or morning stiffness.

Thus according to a fifth aspect, the present invention provides a granular composition as defined herein or a unit dosage form as defined herein for use in medicine.

Accordingly, in a sixth aspect of the present invention there is provided a granular composition as defined herein or a unit dosage form as defined herein for use in the treatment of pain and/or inflammation and/or fever.

According to a seventh aspect there is provided a granular composition or a unit dosage form for use in a method of treating pain and/or inflammation and/or fever comprising the administration of a granular composition as defined herein or a unit dosage form as defined herein to a mammal in need thereof.

According to an eighth aspect, the present invention provides the use of a granular composition as defined herein in the manufacture of a medicament for treating pain and/or inflammation and/or fever.

According to a ninth aspect, the present invention provides the use of a unit dosage form as defined herein in the manufacture of a medicament for treating pain and/or inflammation or fever.

According to a tenth aspect, the present invention provides the use of a molten NSAID for modifying the compressibility of paracetamol. Preferably, the molten NSAID improves the compressibility of paracetamol.

It will be appreciated that all of the features of the first aspect of the present invention represent preferred features of all other aspects of the present invention. Similarly, all of the features of a particular aspect of the present invention represent preferred features of all other aspects of the present invention.

The invention will now be illustrated by the following examples.

In the examples, racemic ibuprofen and flurbiprofen is available from BASF, Germany; naproxen is available from Albemarle Corporation, USA; paracetamol dense powder is available from Mallinckrodt, USA, croscarmellose sodium is available from FMC Corporation, Brussels, Belgium, under the tradename Ac-Di-Sol; French chalk is available from Luzenac, France; colloidal silicon dioxide (also known as colloidal silica) is available from Degussa, Frankfurt, Germany, under the tradename Aerosil 200; magnesium stearate is available from Hays Chemicals UK; stearic acid is available from Hays Chemicals UK; microcrystalline cellulose is available from the FMC Corporation, Brussels, Belgium, under the tradename Avicel PH101; dicalcium phosphate is available from Univar Limited UK under the tradename Emcompress; Lactose NF Fast Flo is available from DMV in Holland; hydroxypropylmethyl cellulose is available from Dow Chemical Company, USA; and Opaspray White is available from Colorcon, USA. The extruder is an APV MPC40 twin-screw extruder available from APV, UK Ltd.

### Dissolution Measurement

The dissolution was measured using the dissolution method described in the US Pharmacopoeia Vol. 23, page 1791, Apparatus 2 using paddles at 50 rpm and a phosphate buffer (selected at pH 7.2 and/or pH 6.0 and/or pH 5.8).

### Crushing Strength

The crushing strength is a measure of the hardness of the tablet. It was measured by recording the diametrical crushing strength when the tablet was broken between the motorised jaws of a Schluniger 6D Tablet Tester. The jaws of the tablet tester were set at the distance settings of 23, 24, 25, 27 and 29. The higher the distance setting the more pressure is applied to the tablet.

In Tables 1, 2 and 4, the values in bold text indicate the components which form part of the melt granular composition and values in normal text indicate the components which form part of the extra-granular composition. The numerical values in these tables refer to the % by wt of each component present in the respective composition.

### Example 1(a): Preparation of the Granular Component

The process for all illustrative examples involves dry blending the paracetamol and NSAID free acid, optionally with other excipients which may be present in the granular component, and then heating the mixture at a temperature of 100 to 165°C in an extruder to melt the NSAID free acid fully and thereby mix the molten NSAID with the paracetamol and other optional excipients. The molten mass is poured onto cooled stainless steel trays or a cooled moving belt at 10 °C and allowed to cool. The molten mixture typically solidifies within 60 seconds; the mixture may be agitated during cooling. The solid mass thus formed is milled by passing through a cone mill having a screen with a round hole of 1mm. The resulting granules are collected.

### Example 1(b): Preparation of a tablet

The respective extra-granular components (shown in normal text in Tables 1, 2 and 4), namely, colloidal silicon dioxide, magnesium stearate, stearic acid, lactose, dicalcium phosphate and microcrystalline cellulose are blended simultaneously with the granular composition formed from Example 1(a) above for approximately 15 minutes in a blender. The dry blended material was fed to a rotary tabletting machine (Fette P1200) and compressed into tablets (machine speed of 100,000 tablets per hour and compaction force of from 6 kN to 25 kN) containing a therapeutic dose of NSAID drug and paracetamol.

### Examples 2 to 8

Tablets were prepared from the components in Table 1 in the same manner as described for Example 1. The compressing weight of each formulation is adjusted to give a tablet containing the desired therapeutic level of NSAID and paracetamol. In Examples 1 to 8 racemic ibuprofen is present in an amount of 200 mg per tablet. Consequently, the paracetamol is present in an amount of 500 mg per tablet for Examples 1 and 4 to 7 and 325 mg per tablet for Examples 2, 3 and 8. However, it will be appreciated that a range of therapeutic doses of NSAID and paracetamol may be employed.

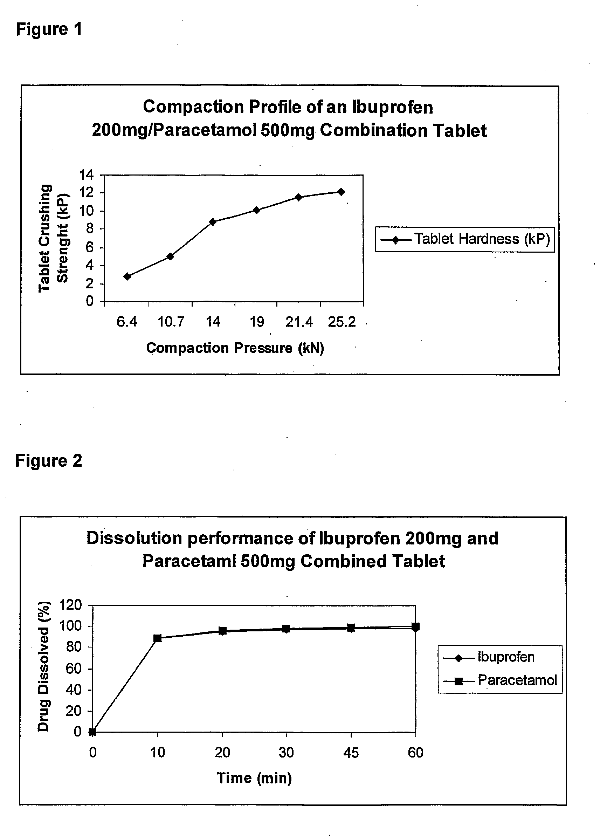

Figure 1 displays the tablet crushing strength versus compaction force applied during the tabletting process for Example 1. In this respect, tablets of acceptable hardness may be formed over a wide range of standard compaction pressures. Suitably, the tablets are sufficiently hard and robust to withstand the further rigours of the manufacturing process i.e. film coating.

### Examples 9 to 16

Tablets were prepared from the components in Table 2 in the same manner as described in Example 1. The compressing weight of each formulation is adjusted to give a tablet containing the desired therapeutic level of NSAID. In this respect, naproxen is present in an amount of 250 mg per tablet in Examples 9 to 12, meloxicam is present in an amount of 20 mg per tablet in Examples 15 and 16, and racemic flurbiprofen is present in an amount of 20 mg per tablet in Examples 15 and 16. Suitably, the paracetamol is present in an amount of 317 mg per tablet in Examples 9 and 11, 488 mg per tablet in Examples 10 and 12, and 237 mg per tablet in Examples 13 to 16.

### Film Coating

The tablets of Examples 1 to 16 may be coated with a film coat composition comprising:

| | % by weight | mg/tablet |
|---|---|---|
| Hydroxypropylmethyl cellulose | 65 | 13 |
| Opaspray White | 21.7 | 4.33 |
| French chalk | 13.3 | 2.67 |

The film coating composition is prepared by dissolving the hydroxypropylmethyl cellulose and Opaspray white in purified water using a Silverson stirrer (Silverson Machines Ltd, UK). French chalk is then dispersed in the resulting solution. The tablet cores are film coated in a conventional perforated pan such as a Manesty Accelacota 150.

In this respect the tablet of Example 1 was coated with the above film coating composition and the dissolution profile of the resulting tablet determined. The dissolution results are depicted in Table 3 and Figure 2.

**Table 1**

| Formulation | % by wt present in formulation | | | | | | | |
|---|---|---|---|---|---|---|---|---|
| | 1 | 2 | 3 | 4 | 5 | 6 | 7 | 8 |
| Racemic Ibuprofen | **23.2** | **29.3** | **29.3** | **23.2** | **26.1** | **26.1** | **24.1** | **29.3** |
| Paracetamol (Acetaminophen) | **58.1** | **47.7** | **47.7** | **58.1** | **65.2** | **65.2** | **60.2** | **47.7** |
| Croscarmellose sodium | **3.4** | **4.4** | **4.4** | **3.4** | **3.1** | **3.1** | **3.3** | **4.4** |
| Microcrystalline cellulose | 13.9 | 17.2 | - | - | 4.7 | - | 11.5 | - |
| Colloidal silicon dioxide | 0.4 | 0.4 | 0.5 | 0.4 | 0.3 | 0.3 | 0.3 | 0.4 |
| Magnesium stearate | 0.5 | 0.5 | 0.5 | 0.5 | 0.3 | 0.3 | 0.3 | 0.4 |
| Stearic acid | 0.5 | 0.5 | 0.5 | 0.5 | 0.3 | 0.3 | 0.3 | 0.3 |
| Lactose Fast Flo | - | - | 17.2 | 13.9 | - | 4.7 | - | - |
| Dicalcium phosphate | - | - | - | - | - | - | - | 17.5 |

| | | | | | | | | |
|---|---|---|---|---|---|---|---|---|
| * Note: Bold indicates the melt phase component | | | | | | | | |

**Table 2**

| Formulation | % by weight present in formulation | | | | | | | |
|---|---|---|---|---|---|---|---|---|
| | 9 | 10 | 11 | 12 | 13 | 14 | 15 | 16 |
| Naproxen*¹ | **37.6** | **28.2** | **37.6** | **28.2** | - | - | - | - |
| Flurbiprofen*² | - | - | - | - | - | - | **4.8** | **4.8** |
| Meloxicam | - | - | - | - | **4.8** | **4.8** | - | - |
| Paracetamol | **47.7** | **55.1** | **47.7** | **55.1** | **57.1** | **57.1** | **57.1** | **57.1** |
| Croscarmellose Sodium | **4.4** | **3.3** | **4.4** | **3.3** | **8.3** | **8.3** | **8.3** | **8.3** |
| Colloidal Silicon Dioxide | 0.4 | 0.3 | 0.4 | 0.3 | 1.0 | 1.0 | 1.0 | 1.0 |
| Magnesium Stearate | 0.4 | 0.4 | 0.4 | 0.4 | - | 1.0 | 1.0 | - |
| Stearic Acid | 0.3 | 0.3 | 0.3 | 0.3 | 1.0 | - | - | 1.0 |
| Microcrystalline Cellulose | 4.4 | 5.5 | - | - | 19.5 | - | 19.5 | - |
| Dixcalcium Phosphate | - | - | - | - | - | - | - | - |
| Lactose NF Fast Flo | - | - | 4.4 | 5.5 | - | 19.5 | - | 19.5 |

| | | | | | | | | |
|---|---|---|---|---|---|---|---|---|
| Note: Bold indicates melt phase component *1 6-Methoxy-alpha-methyl-2-naphthalene acetic acid *2-fluoro-α-methyl-4-biphenyl-acetic acid | | | | | | | | |

**Table 3**

| Time (min) | % by wt ibuprofen based on the total weight of ibuprofen in the tablet | % by wt paracetamol based on the total weight of paracetamol in the tablet |
|---|---|---|
| 0 | 0 | 0 |
| 10 | 89.06 | 88.56 |
| 20 | 95.33 | 96.08 |
| 30 | 97.16 | 98.23 |
| 45 | 98.09 | 99.43 |
| 60 | 98.55 | 100.09 |

The results demonstrate that significant amounts of both ibuprofen and paracetamol are solubilised after only 10 minutes. Suitably, the tablet may provide an increased on-set of therapeutic action.

### Crystal structure of racemic ibuprofen, dense powder paracetamol and solidified extrudate containing ibuprofen, paracetamol and croscarmellose sodium

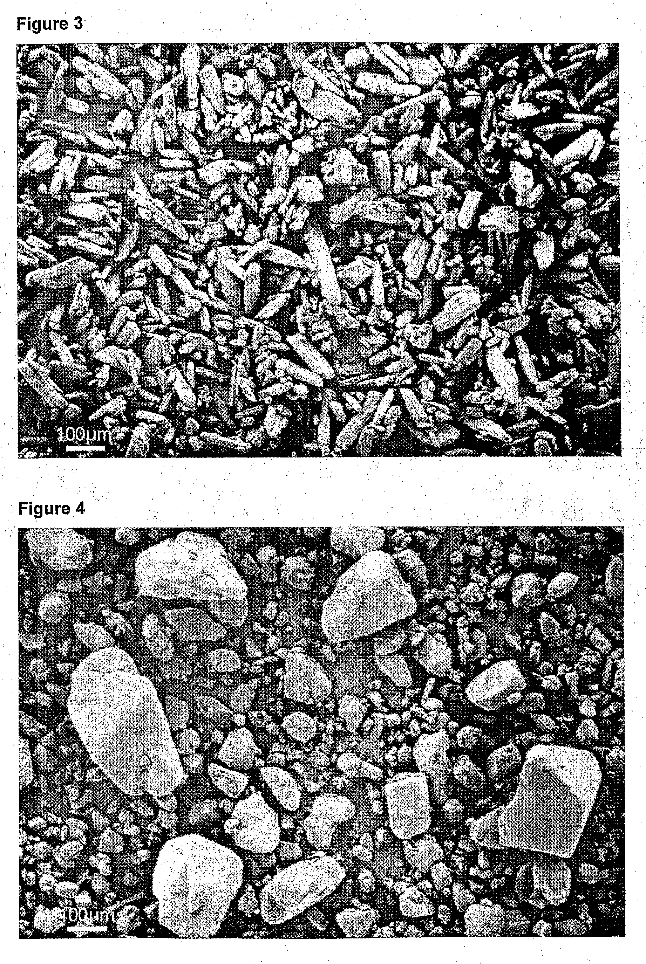

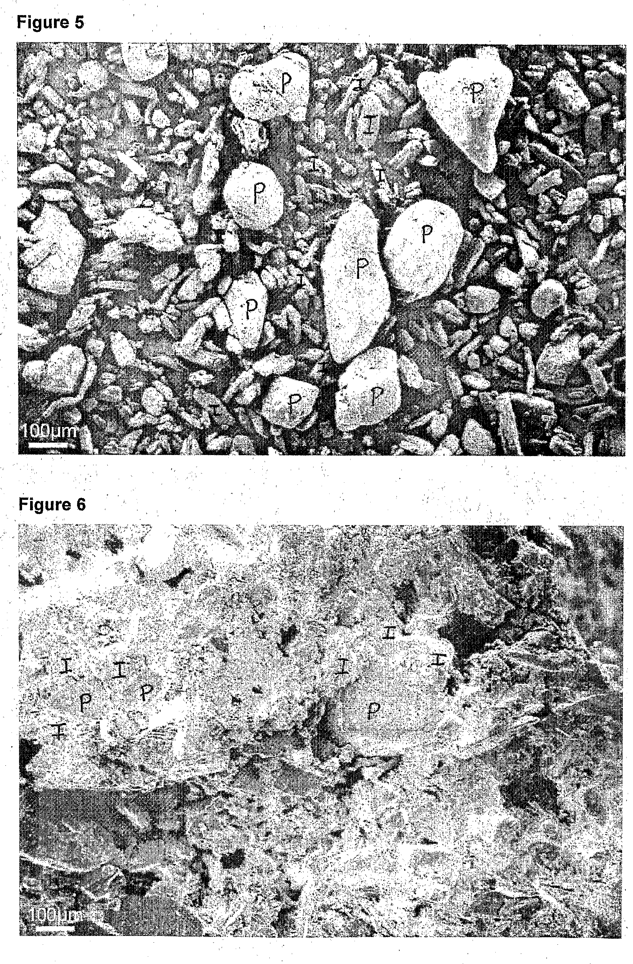

The crystal structures of racemic ibuprofen, dense powder paracetamol, a dry blend of the racemic ibuprofen and dense powder paracetamol, and a solidified extruded mass of racemic ibuprofen and excess paracetamol formed by melting the ibuprofen but not the paracetamol are shown in Figures 3 to 6, respectively. The crystal structures were determined using a scanning electron microscope (SEM).

It is clear from Figures 3, 4 and 5 that paracetamol and ibuprofen have defined crystal structures. The crystals of paracetamol are more irregular in size and shape than the ibuprofen crystals. The paracetamol crystals are essentially cuboid and larger than the ibuprofen crystals which are needle shaped. In Figures 3 and 4 the paracetamol is labelled as P, whereas the ibuprofen is labelled as I. In contrast, the crystal structure of the solidified extruded melt containing an excess amount of paracetamol compared with ibuprofen (Figure 6) shows the paracetamol crystals (P) are essentially distinct, bound together and enrobed by the solidified melt of ibuprofen (I).

### Examples 17 and 18 - Improved flow characteristics and compressability of the pharmaceutical composition of the present invention compared with a dry blend formulation

Tablets comprising racemic ibuprofen and paracetamol Examples 17 and 18 were formed from the formulation as outlined in Table 4.

**Table 4**

| | Example 17 (melt extrusion) | | Example 18 (dry blend) | |
|---|---|---|---|---|
| | % by wt | mg | % by wt | mg |
| Racemic ibuprofen | **30.3** | **200** | 30.3 | 200 |
| Paracetamol | **49.3** | **325** | 49.3 | 325 |
| Croscarmellose sodium | **4.6** | **30** | 4.6 | 30 |
| Anhydrous colloidal silicon dioxide | 0.3 | 2 | 0.3 | 2 |
| Magnesium stearate | 0.4 | 3 | 0.4 | 3 |
| Microcrystalline cellulose | 15.1 | 100 | 15.1 | 100 |

The tablets of Example 17 were formed by the melt extrusion process as described in Examples 1a and 1b. The tablets of Example 18 were formed from a dry blend of the components as listed in Table 3. In particular, the raw materials were sieved via a 16 mesh screen, apart from the anhydrous colloidal silicon dioxide which was passed through a 30 mesh screen, and dry blended together for 15 minutes on a tumble blender. The powder blend was then compressed on a Manesty F3 single punch tablet press using conventional 18 mm x 8 mm caplet tooling to a tablet weight of 660 mg. The mixture exhibited a sticky consistency and showed high levels of sticking to the tablet punches during compression. The mixture was difficult to compress.

In contrast, the formulation of Example 17 as detailed in Table 4 displayed excellent flow characteristics (i.e. non-sticky) and was easier to compress. It was possible to run the rotary tabletting machine at a fast rate of 100,000 tablets per hour.

The crushing strength of the tablets from Examples 17 and 18 was measured by recording the diametrical crushing strength when the tablet was broken between the motorised jaws of a Schluniger 6D Tablet Tester. The crushing strength is a measure of the hardness of the tablet (i.e. a higher crushing strength corresponds to a harder tablet). The jaws of the Tablet Tester were set at a distance setting number of 24, 26, 28 and 29 on the instrument.

The tablets of Example 17 formed by the melt-extrusion process exhibited a crushing strength of 6.9 to 7.1 kP (average of 7.0 kP). Whereas, the tablets of Example 18 formed from the dry blend exhibited a crushing strength of 2.0 to 3.2 kP (average of 2.7 kP).

The results demonstrate that the melt granules used to form the tablets of Example 17 exhibit improved flow characteristics and are easier to compress than the racemic ibuprofen and paracetamol dry blend formulation of Example 18. Moreover, the tablets formed by the melt extrusion process (Example 17) are significantly more robust and harder than corresponding tablets formed from a dry blend mixture (Example 18). Consequently, the tablets of Example 17 are more suited to withstand the rigours of the manufacturing process (i.e. film or sugar coating, packaging etc).

## Claims

1. A process for producing a granular composition comprising a plurality of solidified melt granules including a non-steroidal anti-inflammatory drug (NSAID) and paracetamol, the process comprising the steps of:
(a) forming a melt mixture by mixing a molten NSAID free acid and paracetamol, optionally with one or more excipients; and
(b) forming the melt mixture into solidified melt granules, wherein the paracetamol is present in an amount of greater than or equal to 40% by weight and less than or equal to 80% by weight of the solidified melt granules, based on the total weight of the solidified melt granules.

2. A process as claimed in claim 1 wherein the NSAID is present in an amount of greater than or equal to 20% by weight and less than or equal to 45% by weight of the solidified melt granules, based on the total weight of the solidified melt granules.

3. A process as claimed in claim 1 or claim 2 wherein the ratio by weight of paracetamol to NSAID in the solidified melt granules is greater than or equal to 1.5:1.

4. A process as claimed in claim 3 wherein the ratio by weight of paracetamol to NSAID in the solidified melt granules is greater than or equal to 2:1 and less than or equal to 3:1.

5. A process as claimed in any one of the preceding claims wherein the NSAID free acid is fully molten in the melt-mixture.

6. A process as claimed in any one of the preceding claims wherein the paracetamol does not melt during the formation of the melt mixture.

7. A process as claimed in any one of the preceding claims wherein the NSAID free acid comprises a 2-aryl propionic acid.

8. A process as claimed in claim 7 wherein the 2-aryl propionic acid comprises racemic ibuprofen or S(+)-ibuprofen.

9. A process as claimed in any one of the preceding claims wherein the melt mixture is formed by melt extruding a mixture comprising the NSAID free acid, paracetamol and optionally one or more excipients.

10. A process a claimed in any one of the preceding claims wherein the melt mixture is formed into solidified melt granules by cooling the melt mixture to form a solidified melt and comminuting the solidified melt to form the solidified melt granules.

11. A process as claimed in any one of the preceding claims wherein the melt mixture in step (a) comprises the NSAID free acid, paracetamol and a disintegrating agent.

12. A process as claimed in claim 11 wherein the disintegrating agent is present in an amount of greater than or equal to 1 wt% to less than or equal to 20 wt% of the solidified melt granules based on the total weight of the solidified melt granules.

13. A process as claimed in any one of the preceding claims further including the step of compressing the granular composition to form a non-effervescent compressed tablet.

14. A granular composition comprising a plurality of solidified melt granules comprising a NSAID free acid as defined in any one of claims 1 to 12, paracetamol and optionally one or more excipients as defined in any one of claims 1 to 12, wherein the paracetamol is present in an amount of greater than or equal to 40% by weight and less than or equal to 80% by weight of the solidified melt granules, based on the total weight of the solidified melt granules.

15. A granular composition as claimed in claim 14 wherein the NSAID is present in an amount of greater than or equal to 20% by weight and less than or equal to 45% by weight of the solidified melt granules, based on the total weight of the solidified melt granules.

16. A granular composition as claimed in claim 14 or claim 15 wherein the ratio by weight of paracetamol to NSAID in the solidified melt granules is greater than or equal to 1.5:1.

17. A granular composition as claimed in claim 16 wherein the ratio by weight of paracetamol to NSAID in the solidified melt granules is great than or equal to 2:1 and less than or equal to 3:1.

18. A granular composition as claimed in any one of claims 14 to 17 wherein the solidified melt granules are obtainable by fully melting the NSAID free acid.

19. A granular composition as claimed in any one of the claims 14 to 18 wherein the NSAID free acid comprises a 2-aryl propionic acid.

20. A granular composition as claimed by any one of claims 14 to 19 wherein the 2-aryl propionic acid comprises racemic ibuprofen or S(+)- ibuprofen.

21. A granular composition as claimed in any one of claims 14 to 20 wherein the solidified melt granules comprises a NSAID free acid, paracetamol and a disintegrating agent.

22. A granular composition as claimed in claim 21 wherein the disintegrating agent is present in an amount of greater than or equal to 1 wt% to less than or equal to 20 wt% of the solidified melt granules based on the total weight of the solidified melt granules.

23. A granular composition comprising a plurality of solidified melt granules obtainable by the process as defined in any one of claims 1 to 13.

24. A pharmaceutical composition comprising a unit dosage form comprising a granular composition as defined in any one of claims 14 to 23.

25. A pharmaceutical composition as claimed in claim 24 wherein the unit dosage form comprises a solid unit dosage form, particularly a compressed tablet.

26. A pharmaceutical composition as claimed in claim 24 to 25 wherein the unit dosage form further comprises an extra-granular component.

27. A pharmaceutical composition as claimed in claim 26 wherein the unit dosage form comprises 60 to 99.5% by weight of the granular composition based on the total weight of the unit dosage form.

28. A pharmaceutical composition as claimed in claim 26 to 27 wherein the unit dosage form comprises 0.5 to 40% by weight of the extra-granular component based on the total weight of the unit dosage form.

29. A pharmaceutical composition as claimed in any one of the claims 26 to 28 wherein the extra-granular component includes one or more excipients selected from a diluent, a surfactant, a wicking agent and a lubricant.

30. A pharmaceutical composition as claimed in any one of claims 24 to 29 wherein the unit dosage form includes a further pharmacologically active ingredient.

31. A granular composition as defined in any one of claims 14 to 23 or a pharmaceutical composition as defined in any one of claims 24 to 30 for use in medicine.

32. A granular composition as defined in any one of claims 14 to 23 or a pharmaceutical composition as defined in any one of claims 24 to 30 for use in a treatment of pain and/or inflammation and/or fever.

33. Use of a granular composition as defined in any one of claims 14 to 23 or a pharmaceutical composition as defined in any one of claims 24 to 30 in the manufacture of a medicament for treating pain and/or inflammation and/or fever.

34. Use of a molten NSAID free acid for modifying the compressibility of paracetamol, wherein the paracetamol is present in an amount of greater than or equal to 40% by weight and less than or equal to 80% by weight of solidified melt granules, based on the total weight of the solidified melt granules.

## Patentansprüche

1. Verfahren zum Herstellen einer granulären Zusammensetzung, die eine Vielzahl von verfestigten Schmelzgranulaten umfasst, die ein nicht-steroidales Antirheumatikum (NSAR) und Paracetamol beinhalten, wobei das Verfahren die Schritte umfasst:
(a) Bilden einer Schmelzmischung durch Mischen einer geschmolzenen NSARfreien Säure und Paracetamol, gegebenenfalls mit einem oder mehreren Hilfsstoffen; und
(b) Bilden der Schmelzmischung zu verfestigten Schmelzgranulaten, wobei das Paracetamol in einer Menge von mehr als oder gleich 40 Gew.-% und weniger als oder gleich 80 Gew.-% der verfestigten Schmelzgranulate, bezogen auf das Gesamtgewicht der verfestigten Schmelzgranulate, vorhanden ist.

2. Verfahren nach Anspruch 1, wobei das NSAR in einer Menge von mehr als oder gleich 20 Gew.-% und weniger als oder gleich 45 Gew.-% der verfestigten Schmelzgranulate, bezogen auf das Gesamtgewicht der verfestigten Schmelzgranulate, vorhanden ist.

3. Verfahren nach Anspruch 1 oder Anspruch 2, wobei das Gewichtsverhältnis von Paracetamol zu NSAR in den verfestigten Schmelzgranulaten größer oder gleich 1,5:1 ist.

4. Verfahren nach Anspruch 3, wobei das Gewichtsverhältnis von Paracetamol zu NSAR in den verfestigten Schmelzgranulaten größer oder gleich 2:1 und kleiner oder gleich 3:1 ist.

5. Verfahren nach einem beliebigen der vorhergehenden Ansprüche, wobei die freie NSAR-Säure in der Schmelzmischung vollständig geschmolzen ist.

6. Verfahren nach einem beliebigen der vorhergehenden Ansprüche, wobei das Paracetamol während der Bildung der Schmelzmischung nicht schmilzt.

7. Verfahren nach einem beliebigen der vorhergehenden Ansprüche, wobei die freie NSAR-Säure eine 2-Arylpropionsäure umfasst.

8. Verfahren nach Anspruch 7, wobei die 2-Arylpropionsäure racemisches Ibuprofen oder S (+)-Ibuprofen umfasst.

9. Verfahren nach einem beliebigen der vorhergehenden Ansprüche, wobei die Schmelzmischung durch Schmelzextrudieren einer Mischung gebildet wird, die die NSAR-freie Säure, Paracetamol und gegebenenfalls einen oder mehrere Hilfsstoffe umfasst.

10. Verfahren nach einem beliebigen der vorhergehenden Ansprüche, wobei die Schmelzmischung zu verfestigten Schmelzgranulaten geformt wird, indem die Schmelzmischung gekühlt wird, um eine verfestigte Schmelze zu bilden, und die verfestigte Schmelze zerkleinert wird, um die verfestigten Schmelzgranulate zu bilden.

11. Verfahren nach einem beliebigen der vorhergehenden Ansprüche, wobei die Schmelzmischung in Schritt (a) die freie NSAR-Säure, Paracetamol und ein Zerfallsmittel umfasst.

12. Verfahren nach Anspruch 11, wobei das Zerfallsmittel in einer Menge von mehr als oder gleich 1 Gew.-% bis weniger als oder gleich 20 Gew.-% der verfestigten Schmelzgranulate, bezogen auf das Gesamtgewicht der verfestigten Schmelzgranulate, vorhanden ist.

13. Verfahren nach einem beliebigen der vorhergehenden Ansprüche, ferner umfassend den Schritt des Pressens der granulären Zusammensetzung, um eine gepresste, Nicht-Brausetablette zu bilden.

14. Granuläre Zusammensetzung, umfassend eine Vielzahl von verfestigten Schmelzgranulaten, die eine NSAR-freie Säure nach einem der Ansprüche 1 bis 12, Paracetamol und gegebenenfalls einen oder mehrere Hilfsstoffe nach einem der Ansprüche 1 bis 12 umfassen, wobei das Paracetamol in einer Menge von mehr als oder gleich 40 Gew.-% und weniger als oder gleich 80 Gew.-% der verfestigten Schmelzgranulate, bezogen auf das Gesamtgewicht der verfestigten Schmelzgranulate, vorhanden ist.

15. Granuläre Zusammensetzung nach Anspruch 14, wobei das NSAR in einer Menge von mehr als oder gleich 20 Gew.-% und weniger als oder gleich 45 Gew.-% der verfestigten Schmelzgranulate, bezogen auf das Gesamtgewicht der verfestigten Schmelzgranulate, vorhanden ist.

16. Granuläre Zusammensetzung nach Anspruch 14 oder Anspruch 15, wobei das Gewichtsverhältnis von Paracetamol zu NSAR in den verfestigten Schmelzgranulaten größer oder gleich 1,5:1 ist.

17. Granuläre Zusammensetzung nach Anspruch 16, wobei das Gewichtsverhältnis von Paracetamol zu NSAR in den verfestigten Schmelzgranulaten größer oder gleich 2:1 und kleiner oder gleich 3:1 ist.

18. Granuläre Zusammensetzung nach einem beliebigen der Ansprüche 14 bis 17, wobei das verfestigte Schmelzgranulat durch vollständiges Schmelzen der freien NSAR-Säure erzielbar ist.

19. Granuläre Zusammensetzung nach einem beliebigen der Ansprüche 14 bis 18, wobei die freie NSAR-Säure eine 2-Arylpropionsäure umfasst.

20. Granuläre Zusammensetzung nach einem beliebigen der Ansprüche 14 bis 19, wobei die 2-Arylpropionsäure racemisches Ibuprofen oder S(+)-ibuprofen umfasst.

21. Granuläre Zusammensetzung nach einem beliebigen der Ansprüche 14 bis 20, wobei das verfestigte Schmelzgranulat eine NSAR-freie Säure, Paracetamol und ein Zerfallsmittel umfasst.

22. Granuläre Zusammensetzung nach Anspruch 21, wobei das Zerfallsmittel in einer Menge von mehr als oder gleich 1 Gew.-% bis weniger als oder gleich 20 Gew.-% der verfestigten Schmelzgranulate, bezogen auf das Gesamtgewicht der verfestigten Schmelzgranulate, vorhanden ist.

23. Granuläre Zusammensetzung, umfassend eine Vielzahl von verfestigten Schmelzgranulaten, die nach dem Verfahren gemäß einem der Ansprüche 1 bis 13 erzielbar sind.

24. Pharmazeutische Zusammensetzung, umfassend eine Einheitsdosierungsform, die eine granuläre Zusammensetzung, wie sie in einem der Ansprüche 14 bis 23 definiert ist, umfasst.

25. Pharmazeutische Zusammensetzung nach Anspruch 24, wobei die Einheitsdosierungsform eine feste Einheitsdosierungsform, insbesondere eine gepresste Tablette, umfasst.

26. Pharmazeutische Zusammensetzung nach Anspruch 24 bis 25, wobei die Einheitsdosierungsform ferner eine extragranuläre Komponente umfasst.

27. Pharmazeutische Zusammensetzung nach Anspruch 26, wobei die Einheitsdosierungsform 60 bis 99,5 Gew.-% der granulären Zusammensetzung, bezogen auf das Gesamtgewicht der Einheitsdosierungsform, umfasst.

28. Pharmazeutische Zusammensetzung nach Anspruch 26 bis 27, wobei die Einheitsdosierungsform 0,5 bis 40 Gew.-% der extragranulären Komponente, bezogen auf das Gesamtgewicht der Einheitsdosierungsform, umfasst.

29. Pharmazeutische Zusammensetzung nach einem der Ansprüche 26 bis 28, wobei die extragranuläre Komponente einen oder mehrere Hilfsstoffe beinhaltet, ausgewählt aus einem Verdünnungsmittel, einem oberflächenaktiven Mittel, einem Saugmittel und einem Gleitmittel.

30. Pharmazeutische Zusammensetzung nach einem beliebigen der Ansprüche 24 bis 29, wobei die Einheitsdosierungsform einen weiteren pharmakologisch aktiven Bestandteil beinhaltet.

31. Granuläre Zusammensetzung nach einem beliebigen der Ansprüche 14 bis 23 oder eine pharmazeutische Zusammensetzung nach einem der Ansprüche 24 bis 30 zur Verwendung in der Medizin.

32. Granuläre Zusammensetzung nach einem beliebigen der Ansprüche 14 bis 23 oder eine pharmazeutische Zusammensetzung nach einem der Ansprüche 24 bis 30 zur Verwendung bei der Behandlung von Schmerzen und/oder Entzündungen und/oder Fieber.

33. Verwendung einer granulären Zusammensetzung nach einem beliebigen der Ansprüche 14 bis 23 oder einer pharmazeutischen Zusammensetzung nach einem der Ansprüche 24 bis 30 bei der Herstellung eines Medikaments zur Behandlung von Schmerzen und/oder Entzündungen und/oder Fieber.

34. Verwendung einer geschmolzenen NSAR-freien Säure zum Modifizieren der Komprimierbarkeit von Paracetamol, wobei das Paracetamol in einer Menge von mehr als oder gleich 40 Gew.-% und weniger als oder gleich 80 Gew.-% verfestigter Schmelzgranulate, bezogen auf das Gesamtgewicht der verfestigten Schmelzgranulate, vorhanden ist.

## Revendications

1. Procédé de production d'une composition granulaire comprenant une pluralité de granules fondues solidifiées contenant un médicament anti-inflammatoire non stéroïdien (AINS) et du paracétamol, le procédé comprenant les étapes consistant à :
(a) former un mélange fondu en mélangeant un AINS sous forme d'acide libre, fondu et du paracétamol, éventuellement avec au moins un excipient ; et
(b) transformer le mélange fondu en granules fondues solidifiées, le paracétamol étant présent en une quantité supérieure ou égale à 40 % en poids et inférieure ou égale à 80 % en poids des granules fondues solidifiées, par rapport au poids total des granules fondues solidifiées.

2. Procédé selon la revendication 1, dans lequel l'AINS est présent en une quantité supérieure ou égale à 20 % en poids et inférieure ou égale à 45 % en poids des granules fondues solidifiées, par rapport au poids total des granules fondues solidifiées.

3. Procédé selon la revendication 1 ou la revendication 2, dans lequel le rapport pondéral du paracétamol à l'AINS dans les granules fondues solidifiées est supérieur ou égal à 1,5:1.

4. Procédé selon la revendication 3, dans lequel le rapport pondéral du paracétamol à l'AINS dans les granules fondues solidifiées est supérieur ou égal à 2:1 et inférieur ou égal à 3:1.

5. Procédé selon l'une quelconque des revendications précédentes, dans lequel l'AINS sous forme d'acide libre est entièrement fondu dans le mélange fondu.

6. Procédé selon l'une quelconque des revendications précédentes, dans lequel le paracétamol ne fond pas pendant la formation du mélange fondu.

7. Procédé selon l'une quelconque des revendications précédentes, dans lequel l'AINS sous forme d'acide libre comprend un acide 2-arylpropionique.

8. Procédé selon la revendication 7, dans lequel l'acide 2-arylpropionique comprend de l'ibuprofène racémique ou du S-(+)-ibuprofène.

9. Procédé selon l'une quelconque des revendications précédentes, dans lequel le mélange fondu est formé par extrusion à l'état fondu d'un mélange comprenant l'AINS sous forme d'acide libre, le paracétamol et éventuellement au moins un excipient.

10. Procédé selon l'une quelconque des revendications précédentes, dans lequel le mélange fondu est converti en granules fondues solidifiées par refroidissement du mélange fondu pour former une masse fondue solidifiée et par broyage de la masse fondue solidifiée pour former les granules fondues solidifiées.

11. Procédé selon l'une quelconque des revendications précédentes, dans lequel le mélange fondu à l'étape (a) comprend l'AINS sous forme d'acide libre, du paracétamol et un agent de désintégration.

12. Procédé selon la revendication 11, dans lequel l'agent de désintégration est présent en une quantité supérieure ou égale à 1 % en poids et inférieure ou égale à 20 % en poids des granules fondues solidifiées, par rapport au poids total des granules fondues solidifiées.

13. Procédé selon l'une quelconque des revendications précédentes, comprenant en outre l'étape consistant à comprimer la composition granulaire pour former un comprimé non effervescent obtenu par compression.

14. Composition granulaire comprenant une pluralité de granules fondues solidifiées comprenant un AINS sous forme d'acide libre, tel que défini dans l'une quelconque des revendications 1 à 12, du paracétamol et éventuellement au moins un excipient tel que défini dans l'une quelconque des revendications 1 à 12, le paracétamol étant présent en une quantité supérieure ou égale à 40 % en poids et inférieure ou égale à 80 % en poids des granules fondues solidifiées, par rapport au poids total des granules fondues solidifiées.

15. Composition granulaire selon la revendication 14, dans laquelle l'AINS est présent en une quantité supérieure ou égale à 20 % en poids et inférieure ou égale à 45 % en poids des granules fondues solidifiées, par rapport au poids total des granules fondues solidifiées.

16. Composition granulaire selon la revendication 14 ou 15, dans laquelle le rapport pondéral du paracétamol à l'AINS dans les granules fondues solidifiées est supérieur ou égal à 1,5:1.

17. Composition granulaire selon la revendication 16, dans laquelle le rapport pondéral du paracétamol à l'AINS dans les granules fondues solidifiées est supérieur ou égal à 2:1 et inférieur ou égal à 3:1.

18. Composition granulaire selon l'une quelconque des revendications 14 à 17, dans laquelle les granules fondues solidifiées peuvent s'obtenir par fusion complète de l'AINS sous forme d'acide libre.

19. Composition granulaire selon dans l'une quelconque des revendications 14 à 18, dans laquelle l'AINS sous forme d'acide libre comprend un acide 2-arylpropionique.

20. Composition granulaire selon l'une quelconque des revendications 14 à 19, dans laquelle l'acide 2-arylpropionique comprend de l'ibuprofène racémique ou du S-(+)-ibuprofène.

21. Composition granulaire selon l'une quelconque des revendications 14 à 20, dans laquelle les granules fondues solidifiées comprennent un AINS sous forme d'acide libre, du paracétamol et un agent de désintégration.

22. Composition granulaire selon la revendication 21, dans laquelle l'agent de désintégration est présent en une quantité supérieure ou égale à 1 % en poids et inférieure ou égale à 20 % en poids des granules fondues solidifiées, par rapport au poids total des granules fondues solidifiées.

23. Composition granulaire comprenant une pluralité de granules fondues solidifiées pouvant être obtenues par le procédé tel que défini par l'une quelconque des revendications 1 à 13.

24. Composition pharmaceutique comprenant une forme de dosage unitaire comprenant une composition granulaire telle que définie dans l'une quelconque des revendications 14 à 23.

25. Composition pharmaceutique selon la revendication 24, dans laquelle la forme de dosage unitaire comprend une forme de dosage unitaire solide, en particulier un comprimé obtenu par compression.

26. Composition pharmaceutique selon la revendication 24 ou 25, dans laquelle la forme de dosage unitaire comprend en outre un composant extra-granulaire.

27. Composition pharmaceutique selon la revendication 26, dans laquelle la forme de dosage unitaire comprend 60 à 99,5 % en poids de la composition granulaire, par rapport au poids total de la forme de dosage unitaire.

28. Composition pharmaceutique selon la revendication 26 ou 27, dans laquelle la forme de dosage unitaire comprend 0,5 à 40 % en poids du composant extra-granulaire, par rapport au poids total de la forme de dosage unitaire.

29. Composition pharmaceutique selon l'une quelconque des revendications 26 à 28, dans laquelle le composant extra-granulaire comprend au moins un excipient choisi parmi un diluant, un tensioactif, un agent absorbant et un lubrifiant.

30. Composition pharmaceutique selon l'une quelconque des revendications 24 à 29, dans laquelle la forme de dosage unitaire comprend un autre ingrédient pharmacologiquement actif.

31. Composition granulaire telle que définie dans l'une quelconque des revendications 14 à 23 ou composition pharmaceutique telle que définie dans l'une quelconque des revendications 24 à 30, destinée à un usage médical.

32. Composition granulaire telle que définie dans l'une quelconque des revendications 14 à 23 ou composition pharmaceutique telle que définie dans l'une quelconque des revendications 24 à 30, à utiliser dans le traitement de la douleur et/ou d'une inflammation et/ou de la fièvre.

33. Utilisation d'une composition granulaire telle que définie dans l'une quelconque des revendications 14 à 23 ou d'une composition pharmaceutique telle que définie dans l'une quelconque des revendications 24 à 30 dans la fabrication d'un médicament destiné au traitement de la douleur et/ou Id'une inflammation et/ou de la fièvre.

34. Utilisation d'un AINS sous forme d'acide libre, fondu pour modifier la compressibilité du paracétamol, le paracétamol étant présent en une quantité supérieure ou égale à 40 % en poids et inférieure ou égale à 80 % en poids de granules fondues solidifiées, par rapport au poids total des granules fondues solidifiées.
